# NEW EUROPEAN PATENT SPECIFICATION

(11) **EP 0 894 482 B2**
(45) Date of publication and mention of the opposition decision: **15.09.2010**
(45) Mention of the grant of the patent: 30.03.2005
(21) Application number: 98112611.3
(22) Date of filing: 08.07.1998
(51) Int. Cl.: A61F 5/448

(54) **An ostomy appliance**
Stomavorrichtung
Appareil d'ostéotomie

(30) Priority: 30.07.1997 DK 89997
(43) Date of publication of application: 03.02.1999
(60) Divisional application: 05102343.0
(73) Proprietor: Coloplast A/S, 3050 Humlebaek (DK)
(72) Inventor: Moss, Flemming, 2950 Vedbaek (DK)
(74) Representative: Nilausen, Kim

(56) References cited:
- EP-A- 0 415 282
- WO-A-95/24169
- US-A- 4 834 731
- US-A- 5 607 413
- US-A- 5 618 276

## Description

### FIELD OF THE INVENTION

The invention relates to an ostomy appliance whose proximal side has a convex face which is provided with adhesive by means of which the ostomy appliance can be adhered to the skin of a user, and whose distal side carries a collection bag or a coupling part therefor and a method of producing the same.

### BACKGROUND OF THE INVENTION

In the below, an ostomy patient or an ostomist denotes a person having a colostomy, an ileostomy or a urostomy. In such persons the colon, the ileum or the urethra has been exposed surgically so that the effluents or waste products of the body, which are conveyed through these organs, are discharged through an artificial orifice or opening and are collected in a collection bag, which is usually adhered to the skin by means of an adhesive wafer or plate having an inlet opening for accommodating the stoma.

The production of ostomy products with a flat proximal adhesive side includes producing a plane disc of adhesive suitable for use on the human skin which is provided with a plasticsO sheet on both sides. The plastics sheet on the distal side of the adhesive is attached, e.g. by gluing or welding, to a collection bag or to a coupling part, to which a collection may be detachably coupled. The sheet on the proximal side of the adhesive is a protective sheet, which is removed prior to use, and the ostomy appliance is then adhered to the user's skin by means of the substantially plane proximal side of the adhesive.

In ostomy patients it is frequently seen that the closest surroundings of the stoma, at a distance of 1-2 cm, are recessed or are positioned in a crater or a cavity as compared to the rest of the skin surface surrounding the stoma. For such patients it has been found to be expedient to use an ostomy product where the adhesive surface around the opening for receiving the stoma has a part which is convex and protrudes toward the user in order to enable the adhesive face of the ostomy appliance to engage and adhere to the skin everywhere in the crater or the cavity. In particular, it is important that the ostomy appliance adheres well to the skin in the area next to the stoma in order to give security against deterioration of the adhesive and against leakage. The use of an adhesive wafer having a rigid convex shape may also apply an external pressure to the area next to the stoma which, in particularly in connection with recessed stomas, will ensure a sufficient protrusion of the stoma to aid the discharge of effluents therefrom directly into the collection bag without contacting the exposed adhesive next to the stoma and prolong the time of service of the adhesive wafer or plate. The shape of the protruding part of the adhesive face may e.g. be domed or conical, and such products are known under the designation convex products. Throughout the specification the term convex has this broad meaning irrespective of the actual embodiment.

EP 317 326 describes a convex product having a rigid ring carrying on its entire convex side an intermediate ring of a soft thermoplastic foam material adhered to the rigid ring. The intermediate ring carries a layer of adhesive to position the product on the skin of a user. The foam ring is a cost-adding component, and the attachment by gluing to the rigid ring adds to the cost of the manufacturing process and furthermore, the contact between foam materials and adhesive layers may cause problems if adhesives having a relatively weaker mechanical strength are used.

Danish Patent Application 1529/91 and EP 416 397 describe convex products where a layer of adhesive suitable for use on the human skin is arranged directly on the convex face of a ring-shaped body. In these convex products the convex shape of the adhesive is given by the convex shape of the ring-shaped body, and the adhesive has no possibility of adapting its shape to the user's shape around the stoma. Accordingly, air pockets may occur, which causes deficient adhesion to the user's skin.

US patent No. 5,618,276 discloses an ostomy appliance in which a thermoplastic convex pressure ring also functions as a belt attachment ring. The pressure ring is directly and sealingly secured on its convex side to an adhesive faceplate and it is also directly and sealingly secured on its opposite side to a collection pouch. Only two annular attachment zones, either in the form of heat seals or adhesive seals, are required to secure the pouch, pressure ring and faceplate together. The pressure ring is secured to a pouch along an annular zone of attachment and it is attached to a soft resilient elastomeric backing of an adhesive faceplate by an annular sealing by heat sealing or a hot melt adhesive seal.

WO 95/24169 discloses an ostomy appliance comprising a ring-shaped body having a convex proximal side and a distal side as well as an inner periphery and an outer periphery, a layer of adhesive to attach the ring-shaped body to a user's skin with the proximal side facing the user's skin, which adhesive is present on a thermoplastic carrier sheet having at least the same extent as the adhesive, and that the carrier sheet is disposed between the adhesive and the ring-shaped body and is attached to the proximal side of the ring-shaped body along the inner periphery and the outer periphery thereof with mutual spaces.

US Patent No. 5,607,413 discloses a convex ostomy faceplate with floating flange and a finger recess. Thus, US Patent No. 5,607,413 discloses a convex pressure ring having convex bodyside surface from which an annular integral attachment rib projects which pressure ring has a thin inner connecting portion that extends radially from the rib and and terminates in a stoma-receiving opening. The inner portion is heat sealed to the thermoplastic backing layer of the wafer for the full radial extent of the inner connection portion, and the outer connecting portion of the pressure ring is heat welded to the thermoplastic backing layer of the wafer along annular heat seal zone.

There is neither a disclosure nor an indication of any connection between the ring-shaped body and the carrier sheet between the attachments along the inner periphery and the outer periphery thereof, especially not between the convex surface of the ring-shaped body and the carrier sheet.

Furthermore, the problem to be solved by US Patent No. 5,607,413 lies in providing a relatively simple but highly effective faceplate construction which has both floating flange and convex pressure ring and in which the convex pressure ring contributes significantly in promoting ease of operation of the floating flange.

There is still a risk of leaks if such an ostomy appliance is exposed to stress at the edges as the carrier sheet may not have a sufficient mechanical strength due to stresses built-in if welding is used when the carrier sheet is attached to the proximal side of the ring-shaped body along the inner periphery and the outer periphery manufacture. After a rupture, the risk of a leak is increased considerably.

Thus, there is still a need for an ostomy appliance comprising a ring-shaped body having a convex proximal side and a distal side as well as an inner periphery and an outer periphery, a layer of adhesive to attach the ring-shaped body to a user's skin with the proximal side facing the user's skin, which adhesive is present on a thermoplastic carrier sheet having at least the same extent as the adhesive, and wherein the carrier sheet is disposed between the adhesive and the ring-shaped body and is attached to the proximal side of the ring-shaped body along the inner periphery and the outer periphery thereof and wherein an adhesive layer is present in the area between the inner and outer periphery of the ring-shaped body.

### BRIEF DESCRIPTION OF THE INVENTION

The present invention relates to an ostomy appliance whose proximal side has a convex face which is provided with adhesive by means of which the ostomy appliance can be adhered to the skin of a user, and whose distal side carries a collection bag or a coupling part therefor.

Furthermore, the invention relates to a method for the preparation of such an ostomy appliance.

### BRIEF DESCRIPTION OF THE DRAWINGS

A preferred embodiment of the invention will be explained below with reference to the drawing, in which
fig. 1 is a lateral view of an embodiment of an ostomy product having a coupling part to which a collection bag may be attached,
fig. 2 shows the distal side of the ostomy product of fig. 1,
fig. 3 shows the proximal side of the ostomy product of fig. 1,
fig. 4 shows an axial section through the ostomy product of fig. 1, and
fig. 5 is a lateral view of another embodiment of an ostomy product like in fig. 1, but without a coupling part.

### DETAILED DESCRIPTION OF THE INVENTION

The invention relates to an ostomy appliance according to claim 1.

The carrier sheet may also be attached to the proximal side of the ring-shaped body along the inner pheriphery thereof.

The ostomy appliance of the invention offers the following advantages:

During use, the carrier sheet may be exposed for stress at the edges giving rise to formation of wrinkles which may lead to leakage. Such wrinkling is prevented according to the invention due to the stabilisation of the carrier sheet and the adhesive layer by the adhesive present between the carrier sheet and the ring-shaped body.

It enables the use of an adhesive having a relatively weaker mechanical strength as the there is a reduced risk of formation of wrinkles due to the contact to the ring-shaped body.

Furthermore, the production of the ostomy appliance according to the invention may be facilitated in that when the carrier sheet has been brought into contact with the ring-shaped body, it is secured and may be manipulated without mutual displacement.

An annular welding at the outer part of the carrier sheet gives the following advantages: 1) it ensures the adhering in the area of the ostomy appliance having pulling forces as opposed to the area in vicinity of the ostomy where pressure forces are predominant, 2) the welding assists in fixing the carrier sheet while the adhesive is cooling or hardening or drying, and thus reduces the mould time.

The welded seam at the outer periphery of the ring-shaped body may be annular, but may be interrupted at least in one place, thereby providing for air access, at the outer periphery 14, through the welded seam 32 to the space between the convex, proximal side 11 of the ring-shaped body and the carrier sheet 30. This is advantageous as it prevents trapping of air between the welding seam and the adhesive between the carrier sheet and the ring-shaped body during the manufacture of the appliance of the invention.

If the adhesive has a sufficient strength, the welding may be avoided opening for use of materials not being heat stable or weldable as a convex element, as a carrier sheet and as adhesive. Using a relatively thick layer of a cheaper industrial adhesive having advantageous physical properties (conformability, elasticity) and a carrier sheet may reduce the necessary amount of medical adhesive and reduce the costs. Furthermore, such thicker adhesive layers may follow inward and outward deformation of the carrier sheet caused by forces tending to displace the carrier sheet and also the ring-shaped convex body and, after reduction of such forces tend to recover some of such deformation.

A product according to the invention wherein the adhesive disposed in the area between the inner and outer periphery fills out space therebetween is advantageous in situations where the ostomate needs a higher degree of fixation of the adhesive layer to the convex ostomy appliance in order to fix the skin to reduce wrinkling of the skin which may lead to leaks.

It is preferred that the adhesive disposed in the area between the inner and outer periphery is a hot melt adhesive as hot melt adhesives are often cheap and skin-friendly as they normally do not contain numerous additives for regulating the physical properties thereof.

When thermoforming an adhesive disc with a carrier sheet and a protective sheet to a convex product there is a risk that the thermoplastic sheets when cooled will retract anisotropically with the result that the adhesive disc does not have the desired plane shape, but gets wrinkles and bulges. This may cause poor adhesion to the user's skin.

It is preferred to provide a thermoplastic protective sheet which covers the proximal side of the adhesive, and which has release properties with respect to the adhesive in order to protect the adhesive surface.

In some cases it is preferred to provide a plane layer of a thermostable material which is in close connection with the adhesive, and which extends radially inwards substantially from the outer periphery of the adhesive and overlaps the outer periphery of the ring-shaped body if the carrier sheet needs a support during thermoforming.

An ostomy appliance according to the invention may be prepared by a method which comprises heating at least the central part of a composite disc comprising a layer of adhesive and a carrier sheet, optionally a protective sheet and optionally a paper, said central part being positioned within the outer periphery of the ring-shaped body, deforming the central part so that it is given the desired cross-section corresponding essentially to the form of the ; convex ring-shaped body, optionally placing the ring-shaped body and sealing the ring-shaped body to the adhesive layer and carrier sheet by welding along the outer periphery of the ring-shaped body.

Heating is effected to a temperature at which the adhesive the carrier sheet and the protective sheet become sufficiently plastic, and deformation can take place by vacuum forming or by pressing together the plastic central part of the disc and the ring-shaped body in another manner to cause the desired deformation of the disc.

The deformation may be carried out by vacuum forming, by pressure forming e.g. using pressurised air or a mandrel or directly with the ring-shaped body.

An annular zone of adhesive may be placed on the carrier sheet or on the proximal part of the ring-shaped body. Then, an annular welded seam is made along the outer periphery of the ring-shaped body, optionally together with a heat seal between the carrier sheet and the proximal part of the ring-shaped body. Then, the appliance is cooled and may be removed from the forming tool.

Plane discs of adhesive having a carrier sheet and a protective sheet known per se may also be used for the production of ostomy appliances of the present type, viz. convex products, since the disc of adhesive material having a carrier sheet and a protective sheet may be thermoformed to assume the convex shape, provided that the components have acceptable physical properties.

### DETAILED DESCRIPTION OF THE DRAWINGS

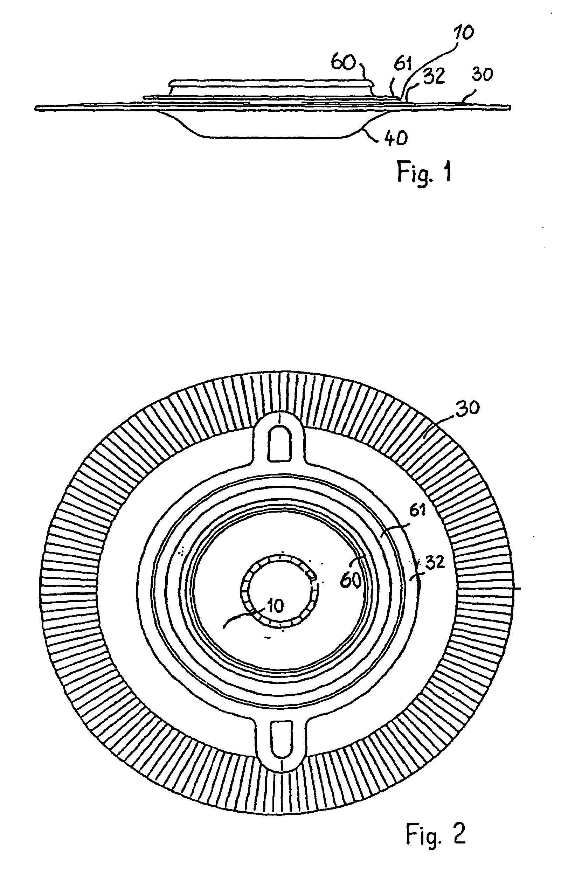

Reference is made to fig. 1 showing an embodiment of an ostomy appliance of the invention having, in direction from the proximal side, a protective sheet or release liner 40 covering the adhesive surface to engage with the skin around a stoma, a carrier sheet 30 for the adhesive layer, a welded seam 32 at the outer periphery of a ring-shaped body 10 for securing the carrier sheet to the ring-shaped body, and a coupling part 60 attached to the ring-shaped body by an annular welded seam 61.

Fig. 2 shows the embodiment of Fig 1 seen from above (from the distal side). In Fig 2 is also showed laterally projecting and diametrically disposed belt-attachment tabs or ears projecting outwardly from opposite edges of the ring-shaped body.

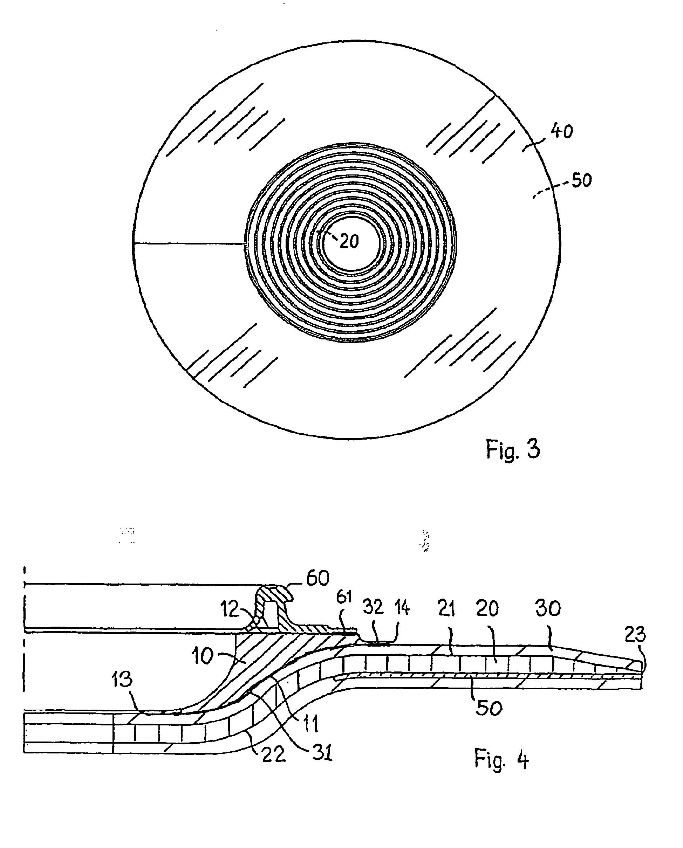

Fig. 3 shows the embodiment of Fig 1 seen from below (from the proximal side)

Fig. 4 shows the ring-shaped body 10 made of a plastics type which is flexible and relatively rigid so that it is substantially dimensionally stable under normally occurring loads. Polyolefins, particularly polyethylene, e.g. in the form of a blend of high and low density polyethylenes, have been found suitable, but other materials having similar properties may be used. The ring-shaped body 10 has a proximal side 11 which is convex, and which forms an S-shaped curve in the shown axial section. Furthermore, the ring-shaped body 10 has a distal side 12 which is plane in the shown embodiment, but the distal side 12 may have other shapes according to the purpose.

A layer 20 of adhesive having essentially the shape of a circular ring disc and consisting of an adhesive suitable for use on the human skin which adhesive is capable of adhering to the skin of a human over an extended period of time. Suitable skin-friendly adhesives include e.g. those disclosed in GB patent specification No. 1 280 631, in DK patent specifications Nos. 127,578, 148,408, 154,806, 147,226 and 154,747, in EP published application Nos. 0 097 846 and 0 415 183, in SE published application No. 365,410, in WO publication No. 88/06894, in US patent specification No. 4,867,748, and in NO published application No. 157,686. Especially preferred are the adhesives disclosed in US patent Nos. 4,367,732 and 5,051,259 and DK patent specification No. 169,711. On its distal side 21 the layer of adhesive 20 is a thermoplastic carrier sheet or backing layer 30, which has substantially the same extent in radial direction as the adhesive 20. The carrier sheet may be made from the same materials as the ring-shaped body. Fig. 4 shows the carrier sheet 30 with the adhesive 20 disposed quite close to the convex proximal side 11 of the ring-shaped body 10, and the carrier sheet 30 is welded to the ring-shaped body 10 along the outer periphery 14 thereof through a welded seam 32. The welded seam 32 at the outer periphery 14 of the ring-shaped body 10 is annular, but may be interrupted at least at one place, thereby providing for air access, at the outer periphery 14, through the welded seam 32 to the space between the convex, proximal side 11 of the ring-shaped body and the carrier sheet 30. Furthermore, a zone of attachment 31 is present in the space between the convex, proximal side 11 of the ring-shaped body and the carrier sheet 30. Said attachment is advantageously in the form of an annular zone of a heat seal or similar or a band of adhesive forming a seal which may cover a part of or all of the proximal side 11 of the ring-shaped body. It is advantageous to use a hot-melt adhesive to form the seal between the ring-shaped body 10 and the carrier sheet 30 or e.g. an adhesive foam material, provided that a liquid tight seal is formed.

On its proximal side the layer of adhesive 20 has a removable protective sheet or release liner 40. The protective sheet or release liner may be formed of a thermoplastic material which has been siliconized on its distal side facing the layer 20 of adhesive, e.g. silicone-coated polyethylene or-silicone-coated paper, enabling an easy removal of the protective sheet prior to use.

A substantially plane layer 50 of paper or another thermostable material which have been treated with a release agent, e.g. siliconized on the side facing the layer of adhesive 20, may be disposed in close connection with the adhesive 20 and extend from the outer periphery 23 thereof and cover the entire plane part of the adhesive 20, said paper layer 50 extending past and further inwards than the outer periphery 14 of the ring-shaped body 10. This stabilises the welded seam 32. The expression thermostable means in this context that the paper layer 50 is stable at the temperatures which are used in the thermoforming of the carrier sheet 30 and the protective sheet 40, between which the adhesive 20 is disposed.

In the production of the ostomy product of figs. 1-4 the starting materials are the ring-shaped body 10 and a plane, composite plate in essentially the shape of a circular ring disc consisting of the layer 20 of adhesive with the carrier sheet 30, adhesive for the zone of attachment 31, the protective sheet 40 and optionally the paper 50.

At least the central part of this composite disc is heated, said central part being positioned within a radius corresponding to the outer periphery 14 of the ring-shaped body 10 as this central part is to be deformed so that it is given the cross-section shown in fig. 4. Heating is effected to a temperature at which the adhesive 20, the carrier sheet 30 and the protective sheet 40 become sufficiently plastic, and deformation can take place by vacuum forming or by pressing together the plastic central part of the disc and the ring-shaped body 10 in another manner to cause the desired deformation of the disc. If desired, an annular zone of adhesive may be placed on the carrier sheet or on the proximal part of the ring-shaped body 10. Then, the annular welded seam 32 is made along the outer periphery 14 of the ring-shaped body 10 optionally together with a heat seal between the carrier sheet and the proximal part (11) of the ring-shaped body 10. In the subsequent cooling, the thermoplastic materials may tend to curl or wrinkle because of an anisotropic contraction, but owing to the presence of the thermostable layer 50, such bulges, wrinkles or curls are avoided, because the thermostable layer of paper is dimensionally stable at the temperatures occurring here.

The distal side 12 of the ring-shaped body 10 is plane, and here a coupling part 60 is attached to the distal side 12 of the ring-shaped body 10 using a ring-shaped welded seam 61. The coupling part 60 is adapted to be coupled to a corresponding coupling part of a collection bag of the ostomy appliance in a manner known per se.

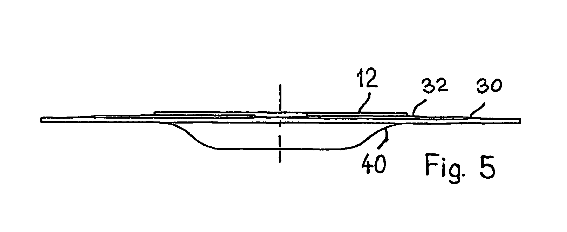

The embodiment of fig. 5 corresponds to the embodiment of figs. 1-4, apart from the absence of a coupling part. In this embodiment, the appliance is adhered to the user's skin and thus forms a substantially plane distal face on which an ostomy product having a plane proximal adhesive face may be attached by adhesion in a manner known per se.

In the alternative, a collection bag may be an integrated part of the ostomy appliance of the invention.

When the ostomy appliance of the invention is to be used, the protective sheet 40 and optionally the paper 50, if present, are removed, thereby exposing the proximal side 22 of the adhesive layer 20, and the ostomy appliance is then adhered to the user's skin such that the appliance is centred around the user's stoma. In the case of a one-piece appliance, a collecting bag is integrated and in the case of a two-piece appliance, a collecting bag is then secured to the ostomy appliance of the invention.

## Claims

1. An ostomy appliance comprising
- a ring-shaped body (10) having a convex proximal side (11) and a distal side (12) as well as an inner periphery (13) and an outer periphery (14),
- a layer of adhesive (20) to attach the ring-shaped body (10) to a user's skin with the proximal side (11) facing the user's skin, said adhesive (20) being present on a thermoplastic carrier sheet (30) having at least the same extent as the adhesive (20), disposed between the adhesive (20) and the ring-shaped body (10)
**characterised in that**, the carrier sheet is attached to the proximal side (11) of the ring-shaped body by a welded seam (32) along the outer periphery (14) thereof, and a further attachment zone (31) is present by an adhesive in the space between the convex (11) proximal side of the ring-shaped body (10) and the carrier sheet (30), and in the area between the inner (13) and outer (14) periphery.

2. An ostomy appliance according to claim 1, **characterised in that** the carrier sheet (30) is attached to proximal side (11) of the ring-shaped body along the inner periphery (13) thereof.

3. An ostomy appliance according to any of the preceding claims, **characterised in that** the adhesive disposed in the area between the inner and outer periphery is a hot melt adhesive.

4. An ostomy appliance according any of the preceding claims, **characterised in that** the adhesive disposed in the area between the inner and outer periphery fills out space there between.

5. An ostomy appliance according to any of the preceding claims, **characterised in that** the carrier sheet (30) is thermoplastic and extends considerably beyond the outer periphery (14) of the ring-shaped body (10), where the carrier sheet (30) is essentially plane.

6. An ostomy appliance according to claim 5, **characterised in that** it comprises a thermoplastic protective sheet (40) which covers the proximal side of the adhesive (20), and which has release properties with respect to the adhesive (20).

7. An ostomy appliance according to claims 5 or 6, **characterised in that** it comprises a plane layer of a thermostable material (50) which is in close connection with the adhesive (20), and which extends radially inwards substantially from the outer periphery (23) of the adhesive and overlaps the outer periphery (14) of the ring-shaped body (10).

## Patentansprüche

1. Stomavorrichtung, die aufweist:
- einen ringförmigen Körper (10) mit einer konvexen proximalen Seite (11) und einer distalen Seite (12) sowie einem Innenumfang (13) und einem Außenumfang (14) und
- eine Schicht eines Klebers (20) zur Befestigung des ringförmigen Körpers (10) auf der Haut eines Anwenders, wobei die proximale Seite (11) zur Haut des Anwenders hin liegt und der Kleber (20) auf einer thermoplastischen Trägerfolie (30), die mindestens die gleiche Ausdehnung wie der Kleber (20) besitzt und zwischen dem Kleber (20) und dem ringförmigen Körper (10) vorgesehen ist, vorliegt,
**dadurch gekennzeichnet, dass** die Trägerfolie an der proximalen Seite (11) des ringförmigen Körpers durch einen verschweißten Rand (32) längs seinem Außenumfang (14) angebracht ist und eine weitere Befestigungszone (31) durch einen Kleber im Bereich zwischen der konvexen proximalen Seite (11) des ringförmigen Körpers (10) und der Trägerfolie (30) und im Bereich zwischen dem Innenumfang (13) und dem Außenumfang (14) vorliegt.

2. Stomavorrichtung nach Anspruch 1, **dadurch gekennzeichnet, dass** die Trägerfolie (30) an der proximalen Seite (11) des ringförmigen Körpers längs seinem Innenumfang (13) befestigt ist.

3. Stomavorrichtung nach einem oder mehreren der vorhergehenden Ansprüche, **dadurch gekennzeichnet, dass** der im Bereich zwischen dem Innenumfang und dem Außenumfang vorgesehene Kleber ein Heißschmelzkleber ist.

4. Stomavorrichtung nach einem oder mehreren der vorhergehenden Ansprüche, **dadurch gekennzeichnet, dass** der im Bereich zwischen dem Innenumfang und dem Außenumfang vorgesehene Kleber den dazwischen liegenden Raum ausfüllt.

5. Stomavorrichtung nach einem oder mehreren der vorhergehenden Ansprüche, **dadurch gekennzeichnet, dass** die Trägerfolie (30) thermoplastisch ist und sich erheblich über den Außenumfang (14) des ringförmigen Körpers (10) hinaus erstreckt, wo die Trägerfolie (30) im Wesentlichen flach ist.

6. Stomavorrichtung nach Anspruch 5, **dadurch gekennzeichnet, dass** sie eine thermoplastische flache Schutzabdeckung (40) aufweist, welche die proximale Seite des Klebers (20) abdeckt und in Bezug auf den Kleber (20) Ablöseeigenschaften besitzt.

7. Stomavorrichtung nach Anspruch 5 oder 6, **dadurch gekennzeichnet, dass** sie eine flache Schicht eines wärmebeständigen Materials (50) aufweist, die in enger Verbindung mit dem Kleber (20) steht und sich im Wesentlichen vom Außenumfang (23) des Klebers radial nach innen erstreckt und den Außenumfang des ringförmigen Körpers (10) überlappt.

## Revendications

1. Accessoire de stomie comprenant
- un corps de forme annulaire (10) ayant une face proximale convexe (11) et une face distale (12) ainsi qu'une périphérie interne (13) et une périphérie externe (14),
- une couche d'adhésif (20) pour fixer le corps de forme annulaire (10) sur la peau d'un utilisateur avec la face proximale (11) au contact de la peau de l'utilisateur, ledit adhésif (20) se trouvant sur une pellicule de support thermoplastique (30) présentant au moins les mêmes dimensions que l'adhésif (20), disposée entre l'adhésif (20) et le corps de forme annulaire (10)
**caractérisé en ce que** la pellicule de support est fixée sur la face proximale (11) du corps de forme annulaire par un soudage en continu (32) le long de la périphérie externe (14) de celui-ci et une autre zone de fixation (31) est présente au moyen d'un adhésif disposé dans l'espace entre la face proximale (11) convexe du corps de forme annulaire (10) et la pellicule de support (30) et dans la zone entre la périphérie interne (13) et la périphérie externe (14).

2. Accessoire de stomie selon la revendication 1, **caractérisé en ce que** la pellicule de support (30) est fixée sur la face proximale (11) du corps de forme annulaire le long de la périphérie interne (13) de celui-ci.

3. Accessoire de stomie selon l'une quelconque des revendications précédentes, **caractérisé en ce que** l'adhésif disposé dans la zone entre la périphérie interne et la périphérie externe est un adhésif thermofusible.

4. Accessoire de stomie selon l'une quelconque des revendications précédentes, **caractérisé en ce que** l'adhésif disposé dans la zone entre la périphérie interne et la périphérie externe comble l'espace entre les deux.

5. Accessoire de stomie selon l'une quelconque des revendications précédentes, **caractérisé en ce que** la pellicule de support (30) est thermoplastique et s'étend considérablement au-delà de la périphérie externe (14) du corps de forme annulaire (10), où la pellicule de support (30) est essentiellement plane.

6. Accessoire de stomie selon la revendication 5, **caractérisé en ce qu'**il comprend une pellicule de protection thermoplastique (40) qui recouvre la face proximal de l'adhésif (20) et qui possède des propriétés de séparation vis-à-vis de l'adhésif (20).

7. Accessoire de stomie selon les revendications 5 ou 6, **caractérisé en ce qu'**il comprend une couche plane constituée d'un matériau thermostable (50) qui est en contact étroit avec l'adhésif (20) et qui s'étend radialement vers l'intérieur sensiblement à partir de la périphérie externe (23) de l'adhésif et recouvre la périphérie externe (14) du corps de forme annulaire (10).
